# EUROPEAN PATENT APPLICATION

(11) **EP 1 754 499 A1**
(43) Date of publication of application: **21.02.2007**
(21) Application number: 05018060.3
(22) Date of filing: 19.08.2005
(51) Int. Cl.: A61M 5/50, A61M 5/34

(54) **Safety hypodermic syringe with retractable needle carrier**

(71) Applicant: Wu, Wei-Shui, Taichung Hsien (TW)
(72) Inventor: Wu, Wei-Shui, Taichung Hsien (TW)
(74) Representative: Viering, Jentschura & Partner

(57) **Abstract**

A safety hypodermic syringe with retractable needle carrier (3) and a hypodermic needle (36) connected therewith is disclosed. The syringe of the present invention comprises a barrel (1), a plunger (2), a needle carrier (3) and a needle cap (4). Several engagement members (34) are provided on the outside of the needle carrier (3), and several engagement members (14) are provided on the inside of the barrel (1); the engagement members (34) of the needle carrier (3) may engage with the engagement members (14) of the barrel (1) so that the needle carrier (3) may be securely held at the distal end of the barrel (1). Several positioning members (33) are provided on the inner side of the needle carrier (3), and several positioning members (22) are provided on the distal end of the plunger (2); the positioning members (33) of the needle carrier (3) may engage with the positioning members (22) of the plunger (2) so that the needle carrier (3) may be connected with the plunger (2). A break-apart member (16) and a neck portion (24) allow a user to break the plunger (2) apart. Hence, a user may first use the plunger (2) to position the needle carrier (3) in place, administrate an injection, pull the plunger (2) backwards to retract the needle carrier (3) and keep the needle carrier (3) inside the barrel (1) so that no person will have a direct contact with the hypodermic needle (36).

## Description

### BACKGROUND OF THE INVENTION

### 1. Field of the invention

The invention generally relates to a safety hypodermic syringe. More particularly, the invention relates to a safety hypodermic syringe with a needle carrier which may be retracted into the barrel and the plunger may be broken apart after use.

### 2. DESCRIPTION OF THE PRIOR ART

As of now, with the advancement of medical technology, utmost importance has been placed on the safety in medical practices. The use of medical devices, especially invasive medical devices, with poor design may cause the patients to catch various diseases. For example, a hypodermic syringe with a poor design may cause irreversible tragedies.

To ensure the high quality in medical practices, the focus should be put in the development of medical devices in addition to the proper medical procedures. As of now, regarding the use of hypodermic syringe, the most important preventive measures are the avoidance of the repeated use of a syringe and the unwanted direct contact with the needle. To avoid the repeated use, all used syringes must be rendered unusable, be placed in a sealed container/bag and then be incinerated; however, the rendering of the syringes into an unusable condition requires additional labor; hence, many medical institutions or clinics only separate the needle from the barrel; therefore, it is possible that such used syringes may be reused. In addition, though the relevant medical persons may wear a pair of gloves when rendering used syringes unusable, they may still be stuck by a needle and then catch a disease.

Hence, a safety hypodermic syringe should allow the needle to be retracted into a safe position after it is used to avoid unwanted direct contact with the needle when a person in the medical profession tries to remove the needle from the barrel.

The inventor of the present invention has been awarded with several patents on his prior inventions; however, these prior inventions have one or more disadvantages (such as complicated design and weak connection between the plunger and the needle carrier). Hence, the inventor put in more effort and has come up with the present invention in which these disadvantages are removed.

### SUMMARY OF THE INVENTION

An object of the present invention is to provide a safety hypodermic syringe so that safety in use may be achieved, undesirable contact with the hypodermic needle may be avoided and the goal of environmental friendliness may be reached.

Another object of the present invention is to provide a safety hypodermic syringe which allows the needle cap to be inserted into the barrel after use.

The third object of the present invention is to provide a safety hypodermic syringe which allows the needle and the needle carrier to be contained in the fluid space of the barrel after use.

The fourth object of the present invention is to provide a safety hypodermic syringe which allows a user to move the plunger to the right and to the left for several times after using the syringe so as to break a neck portion apart from the rest of the plunger.

To reach these objects, the safety hypodermic syringe of the present invention comprises a barrel, a plunger, a needle carrier and a needle cap. The barrel has a fluid space; a positioning space is provided at the distal end of the fluid space; a hole is provided on the distal surface of the barrel; a sloped circular surface is provided so that the distal portion of the barrel has the configuration of a truncated cone; a plurality of engagement members are provided in the positioning space and each of the engagement members has one or two sloped guiding surfaces; a break-apart member is provided at the proximal end of the fluid space. The plunger is arranged inside of the barrel; a top portion is provided at the distal end of the plunger; a plurality of positioning members are provided on the top portion; a groove is provided behind the positioning members; a circular bung is arranged on the groove; a neck portion is provided behind the circular bung on the plunger and may be broken apart from the rest of the plunger.

The needle carrier is arranged in the positioning space of the barrel; a passage is provided in the needle carrier; a needle may be fitted in the passage via an adhesive or friction; a space is provided behind the passage; a plurality of positioning members are provided in the space; a plurality of engagement members are provided on the outside of the needle carrier; a sloped circular surface is provided on the needle carrier and may have a tight fit with the sloped circular surface of the barrel. The needle cap has a cylindrical body with a dome-shaped tip and may enclose the needle.

In use, a user first places the needle cap on the needle and then pushes the plunger forwards to engage the positioning members of the needle carrier with the positioning members of the plunger. Next, the user pushes the plunger forwards further to move the needle out of the barrel through the hole. Then, the user rotates the plunger to engage the engagement members of the needle carrier with the engagement members of the barrel. Then, the user may take the needle cap off and insert the needle into a medication container (not shown) to draw the medication. Now, the user may administrate an injection. After the injection is completed, the user may push the plunger forwards to ensure a secured engagement between the positioning members of the plunger and the positioning members of the needle carrier; then, the user may rotate the plunger and the needle carrier in the opposite direction to disengage the engagement members of the needle carrier from the engagement members of the barrel. Next, the user may pull the plunger backwards in the fluid space until the needle carrier is seized by the break-apart member; now, the user may move the plunger to the right and to the left for several times to break the neck portion apart from the rest of the plunger. Now, the circular bung remains inside the barrel so as to seal off the barrel. Finally, the user may insert the needle cap in an inverted manner into the hole as to block the hole. Now, the safety hypodermic syringe is ready to be discarded.

### BRIEF DESCRIPTION OF THE DRAWINGS

The drawings disclose an illustrative embodiment of the present invention which serves to exemplify the various advantages and objects hereof, and are as follows:
Fig. 1 is a perspective exploded view of a preferred embodiment of the present invention.
Fig. 2A is a perspective view of a first example of the plunger.
Fig. 2B is a perspective view of a second example of the plunger.
Fig. 3A is a side view of a first example of the needle carrier.
Fig. 3B is a sectional view of the first example of the needle carrier.
Fig. 3C is a perspective view of the first example of the needle carrier.
Fig. 3D is a sectional view of a second example of the needle carrier.
Fig. 4 is a perspective view showing the engagement of the plunger and the needle carrier.
Fig. 5 is a perspective view showing the needle cap is placed on the needle carrier and the needle carrier is connected with the plunger inside the barrel.
Fig. 6 is a perspective view showing the engagement members (the first example) of the needle carrier engages with the engagement members (the first example) of the barrel.
Fig. 6A is a partially enlarged perspective view of Fig. 6.
Fig. 6B is a partially enlarged perspective view showing the engagement members (the second example) of the needle carrier engages with the engagement members (the second example) of the barrel.
Fig. 6C is a partially enlarged perspective view showing the engagement members (the second example) of the needle carrier engages with the engagement members (the third example) of the barrel.
Fig. 7 is a perspective view showing the medication is extracted from the medication container and then is stored in the fluid space.
Fig. 8 is a perspective view showing a user rotates the plunger (the first example) and the needle carrier in the opposite direction to disengage the engagement members of the needle carrier from the engagement members of the barrel.
Fig. 9 is a perspective view showing the plunger (the first example) is broken apart and a portion of the plunger and the needle carrier remain inside the barrel.

### DETAILED DESCRIPTION OF THE PREFERRED EMBODIMENT

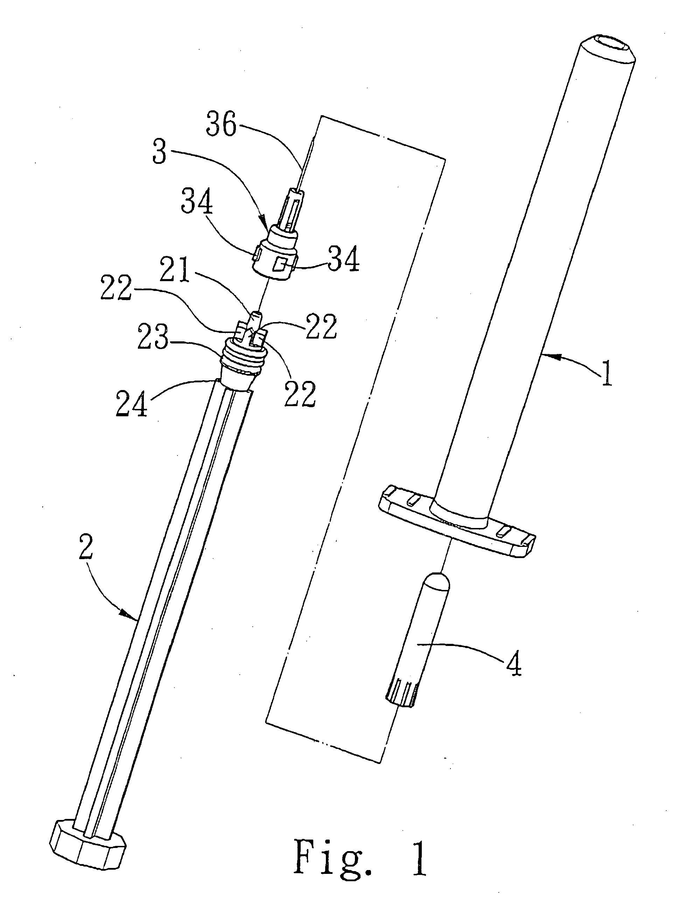

Referring to Fig. 1, the safety hypodermic syringe of the present invention comprises a barrel 1, a plunger 2, a needle carrier 3 and a needle cap 4.

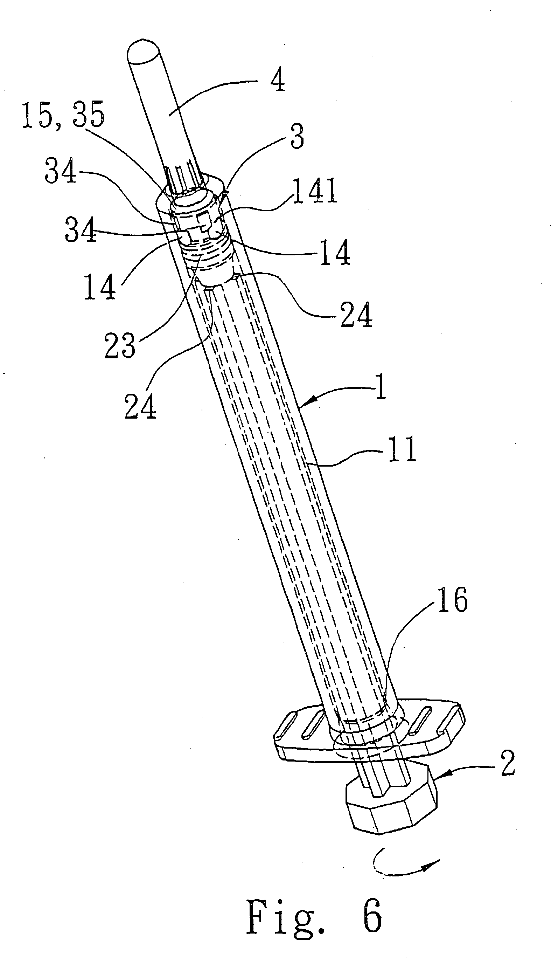

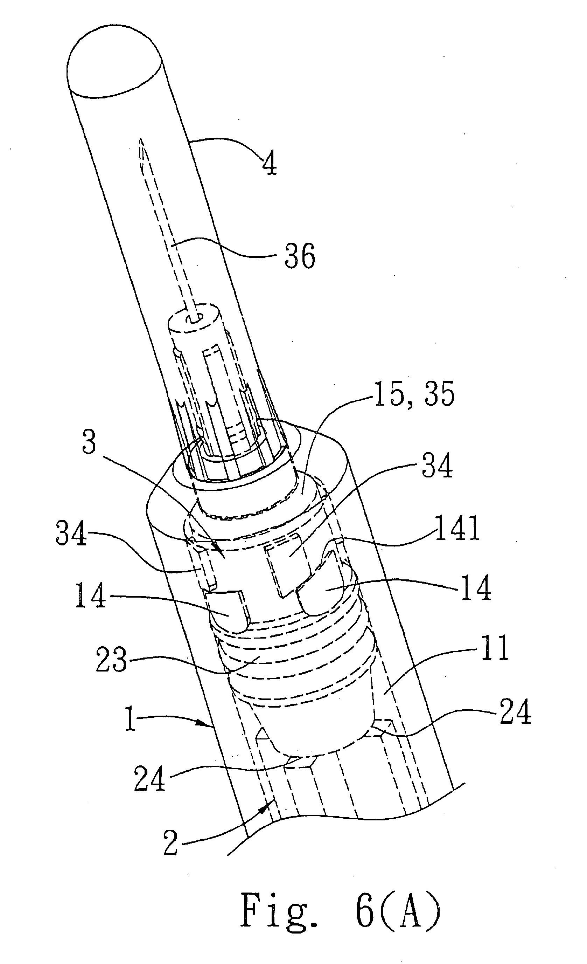

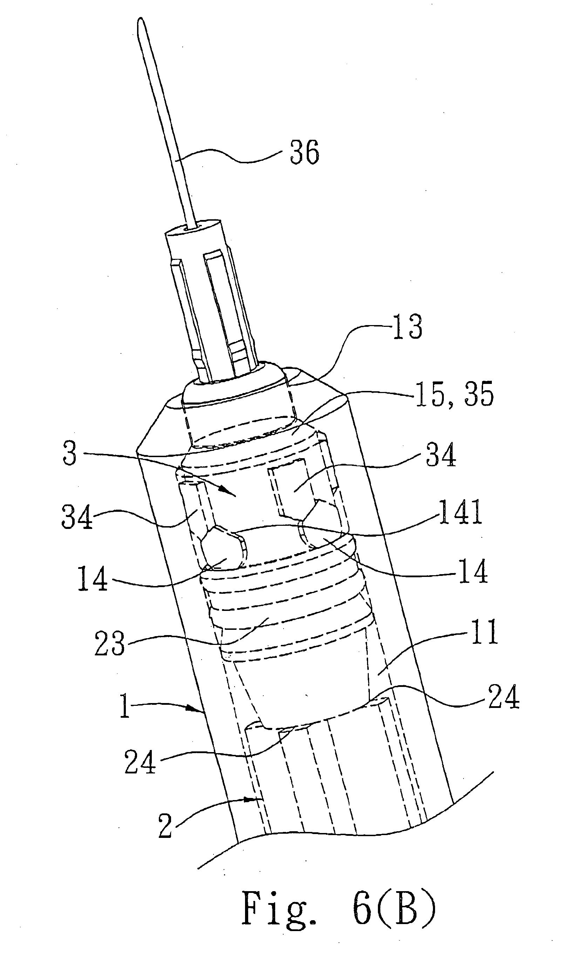

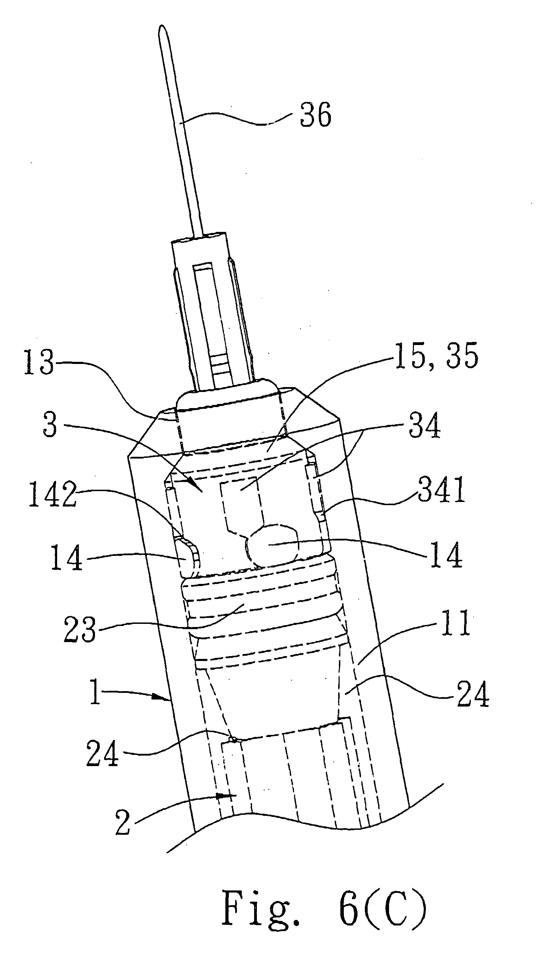

The barrel 1 has a fluid space 11, which may hold medication. A positioning space 12 is provided at the distal end of the fluid space 11 and may fixedly hold the needle carrier 3. A hole 13 is provided on the distal surface of the barrel 1, so as to allow a needle 36 of the needle carrier 3 to move out of the barrel 1. A plurality of engagement members 14 are provided in the positioning space 12. These engagement members 14 are of the same shape and size and are circumferentially (equally) spaced apart in the positioning space 12. Each of the engagement members 14 has one or two sloped guiding surfaces 141 (as illustrated in Fig. 6B) so that the needle carrier 3 may engage with the engagement members 14. Alternatively, each of the engagement members 14 has an arc-shaped guiding surface 142 (as illustrated in Fig. 6C). A sloped circular surface 15 is provided so that the distal portion of the barrel 1 has the configuration of a truncated cone. A break-apart member 16 is provided behind the fluid space 11 and may be used to break the plunger 2 apart.

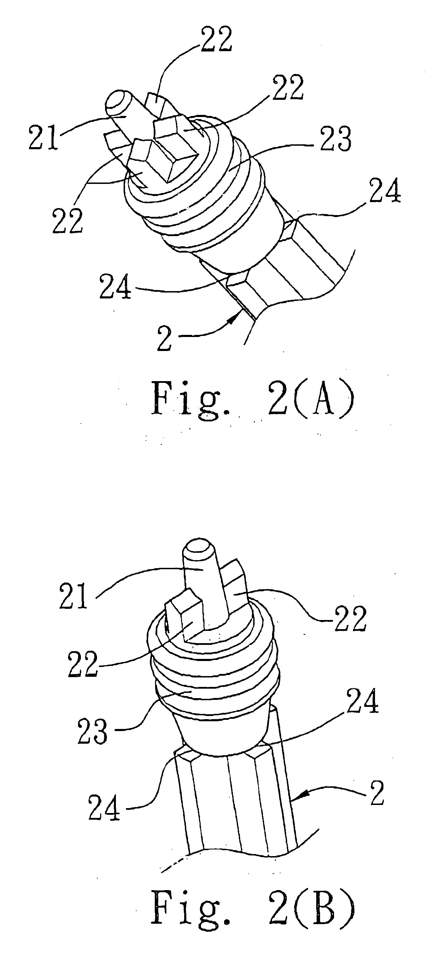

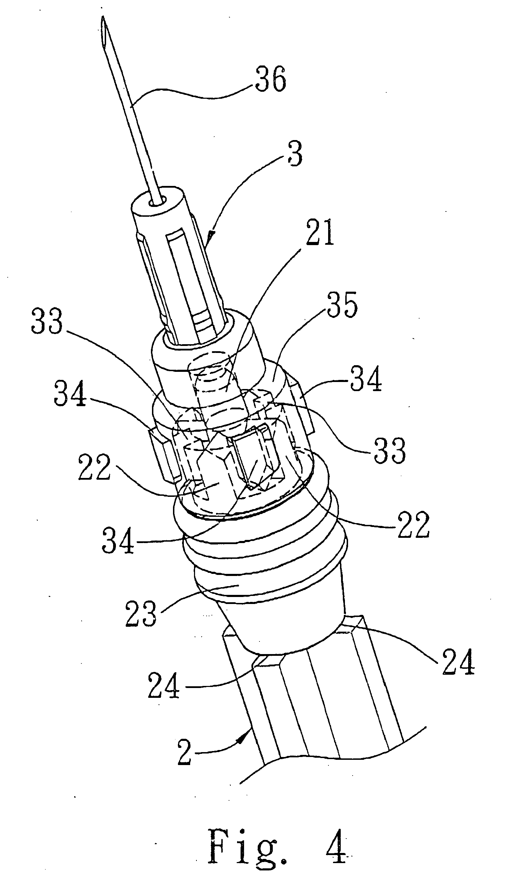

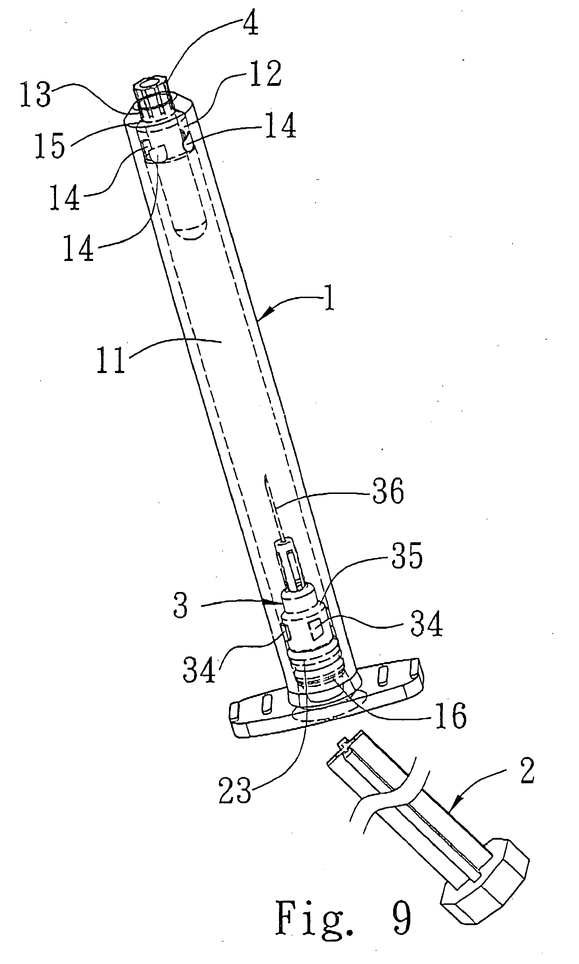

The plunger 2 is arranged inside of the barrel 1 and may be used to move the medication out of the barrel 1. A top portion 21 is provided at the distal end of the plunger 2. A plurality of positioning members 22 are provided on the top portion 21, as illustrated in Fig. 2A and 2B. These positioning members 22 are of the same shape and size and are (equally) spaced apart so as to allow the plunger to connect with the needle carrier 3, as illustrated in Fig. 4. A groove is provided behind the positioning members 22, and a circular bung 23 is provided on the groove. The circular bung 23 is made of rubber or silicone rubber or other soft rubber material and may create a hermetic seal in the fluid space 11. A neck portion 24 is provided behind the circular bung 23 and allows the plunger 2 to be broken apart, as illustrated in Fig. 9 (but keeps the circular bung 23 in the barrel 1).

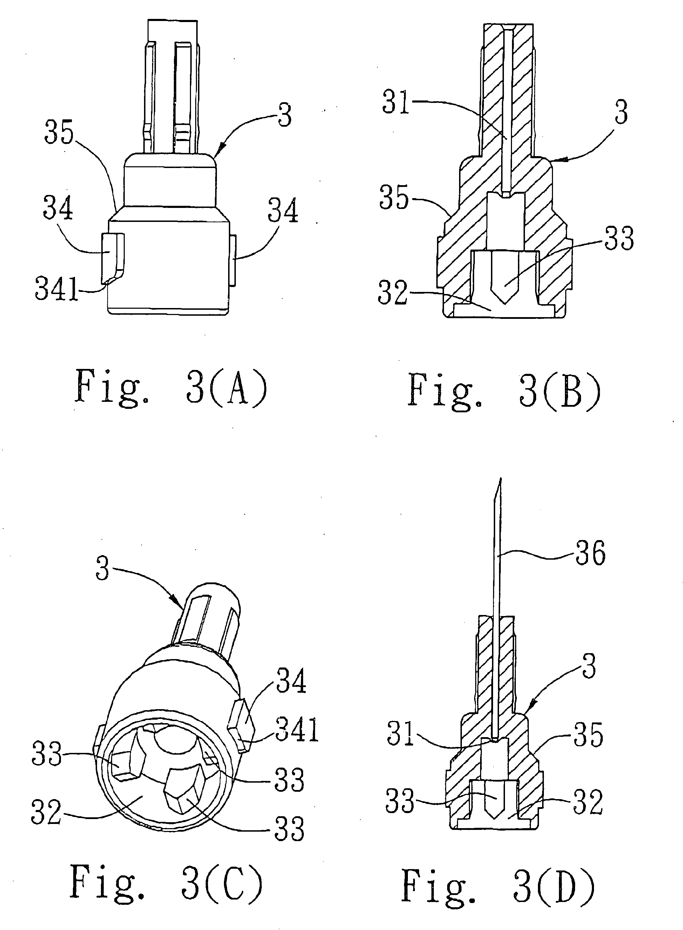

The needle carrier 3 is arranged in the positioning space 12 of the barrel 1. A passage 31 is provided in the needle carrier 3, as shown in Fig. 3B. The needle 36 is fitted in the passage 31 via an adhesive or friction, as illustrated in Fig. 3D. In use, the needle may be moved out of the barrel 1 through the hole 13. A space 32 is provided behind the passage 31. A plurality of positioning members 33 are provided in the space 32, as illustrated in Fig. 3C. These positioning members 33 are of the same shape and size and are (equally) spaced apart. In use, these positioning members 33 may engage with the positioning members 22 of the plunger 2. A plurality of engagement members 34 are provided on the outside of the needle carrier 3, as illustrated in Fig. 3A. These engagement members 34 are of the same shape and size and are circumferentially (equally) spaced apart on the needle carrier 3. Each of the engagement members 34 has one or two sloped guiding surfaces 341 (as illustrated in Fig. 6B) so that the engagement members 34 may engage with the engagement members 14 of the barrel 1 via circumferential movement.

The needle cap 4 has a cylindrical shape and is used to enclose the needle.

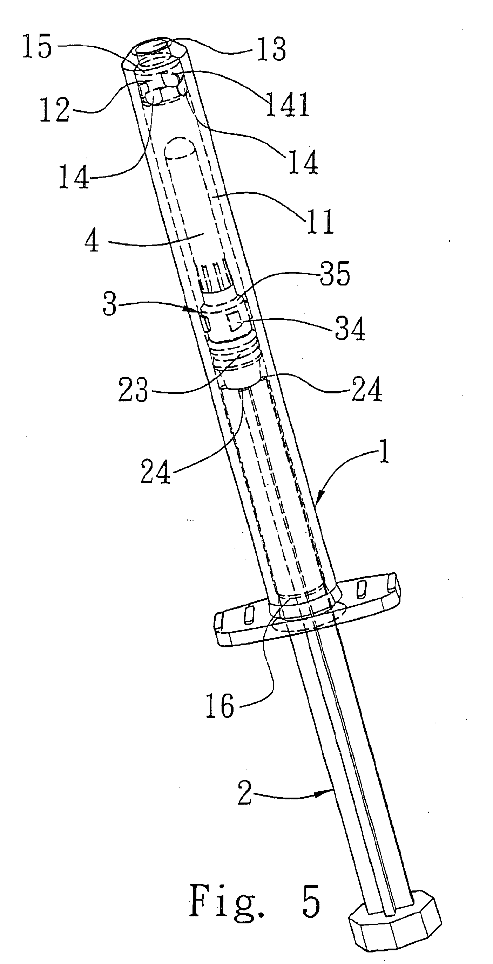

Now, please refer to Fig. 5. When the safety hypodermic syringe of the present invention is used, a user first places the needle cap 4 on the needle and then pushes the plunger 2 forwards to engage the positioning members 33 of the needle carrier 3 with the positioning members 22 of the plunger 2. Next, the user pushes the plunger 2 forwards further to move the needle 36 out of the barrel 1 through the hole 13. Then, the user rotates the plunger 2 so that the engagement members 34 of the needle carrier 3 may engage with the engagement members 14 of the barrel 1, as illustrated in Fig. 6 and 6A. Also, sloped circular surfaces 15 and 35 provided in the positioning space 12 and outside the needle carrier 3, respectively, allow a tight fit between the barrel 1 and the needle carrier 3 and so that no gap remains in the fluid space 11 of the barrel 1.

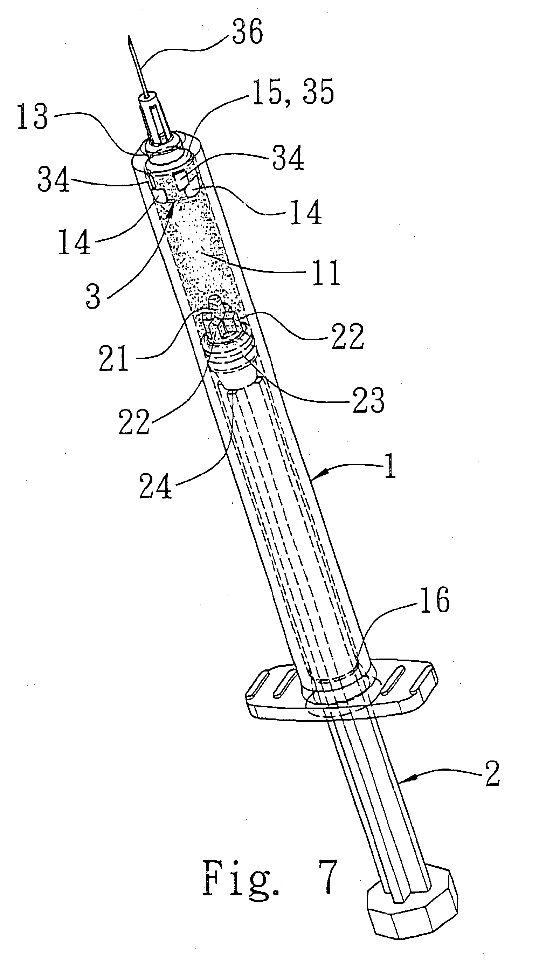

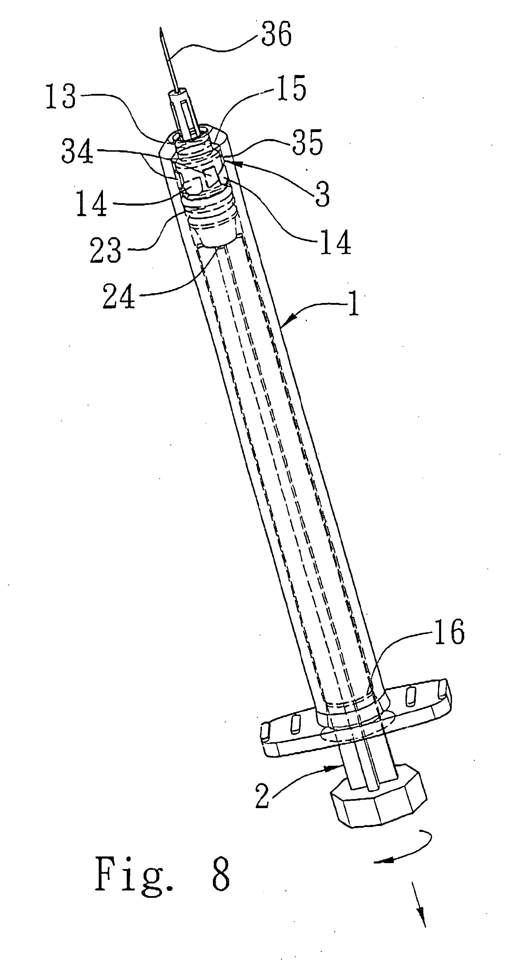

Then, the user may take the needle cap 4 off and insert the needle 36 into a medication container (not shown). Then, the user may pull the plunger 2 backwards. Because the needle carrier 3 is securely seated in the barrel 1, the positioning members 22 of the plunger 2 may be easily disengaged from the positioning members 33 of the needle carrier 3 and the medication may be drawn from the medication container and then be stored in the fluid space 11, as illustrated in Fig. 7. When the fluid space 11 is filled with the medication, injection may then be administrated. After the injection is completed, the user may push the plunger 2 forwards to engage the positioning members 22 of the plunger 2 with the positioning members 33 of the needle carrier 3; then, the user may rotate the plunger 2 and the needle carrier 3 in the opposite direction to disengage the engagement members 34 of the needle carrier 3 from the engagement members 14 of the barrel 1, as illustrated in Fig. 8. Next, the user may pull the plunger backwards in the fluid space 11 until the needle carrier 3 is seized by the break-apart member 16; now, the user may move the plunger 2 to the right and to the left for several times to break the neck portion 24 apart from the rest of the plunger 2, as illustrated in Fig. 9. Now, the circular bung 23 remains inside the barrel 1 so as to seal off the barrel 1. Finally, the user may insert the needle cap 4 in an inverted manner into the hole 13 as to block the hole. Now, the safety hypodermic syringe is ready to be discarded.

In contrast to the aforesaid prior inventions of the inventor and similar devices of the prior art, the safety hypodermic syringe of the present invention has the following advantages:
1. Guiding surfaces are provided on the engagement members of the barrel and the engagement members of the needle carrier so that the needle carrier may be securely held inside the barrel.
2. The positioning members of the plunger may engage with the positioning members of the needle carrier, and a user may rotate the plunger and the needle carrier in the opposite direction to disengage the engagement members of the needle carrier from the engagement members of the barrel.
3. The diameter of the needle cap is slightly larger than that of the hole. A user may insert the needle cap in an inverted manner into the hole so as to block the hole.

Many changes and modifications in the above described embodiment of the invention can, of course, be carried out without departing from the scope thereof. Accordingly, to promote the progress in science and the useful arts, the invention is disclosed and is intended to be limited only by the scope of the appended claims.

## Claims

1. A safety hypodermic syringe with retractable needle carrier, comprises:
a barrel, the barrel having a fluid space, a positioning space being provided at the distal end of the fluid space, a hole being provided on the distal surface of the barrel, a sloped circular surface being provided in the positioning space so that the distal portion of the barrel has the configuration of a truncated cone, a plurality of engagement members being provided in the positioning space, each of the engagement members having one or two sloped guiding surfaces;
a plunger, the plunger being arranged inside of the barrel, a top portion being provided at the distal end of the plunger, a plurality of positioning members being provided on the top portion, a groove being provided behind the positioning members, a circular bung being arranged on the groove and creating a hermetic seal in the fluid space; and
a needle carrier, the needle carrier being arranged in the positioning space of the barrel, a passage being provided in the needle carrier, a space being provided behind the passage, a plurality of positioning members being provided in the space and engaging with the positioning members of the plunger, a plurality of engagement members being provided on the outside of the needle carrier, each of the engagement members has one or two sloped guiding surfaces so that the engagement members may engage with the engagement members of the barrel, each of the engagement members having one or two sloped guiding surfaces, a sloped circular surface being provided on the needle carrier and having a tight fit with the sloped circular surface of the barrel.

2. The safety hypodermic syringe as in claim 1, wherein the needle may be enclosed by a needle cap.

3. The safety hypodermic syringe as in claim 1, wherein each of the engagement members of the barrel has one sloped guiding surface.

4. The safety hypodermic syringe as in claim 1, wherein each of the engagement members of the barrel has two sloped guiding surfaces.

5. The safety hypodermic syringe as in claim 1, wherein each of the engagement members of the barrel has an arc-shaped guiding surface.

6. The safety hypodermic syringe as in claim 1, wherein the engagement members of the barrel are circumferentially (equally) spaced apart in the positioning space.

7. The safety hypodermic syringe as in claim 1, wherein the positioning members of the plunger are provided on the top portion and are (equally) spaced apart.

8. The safety hypodermic syringe as in claim 1, wherein a needle is fitted in the passage via an adhesive or friction.

9. The safety hypodermic syringe as in claim 1, wherein the positioning members of the needle carrier are provided in the space and are (equally) spaced apart.

10. The safety hypodermic syringe as in claim 1, wherein the engagement members of the needle carrier are provided on the outside of the needle carrier and are circumferentially (equally) spaced apart.

11. The safety hypodermic syringe as in claim 1, wherein each of the engagement members of the needle carrier has one sloped guiding surface.

12. The safety hypodermic syringe as in claim 1, wherein each of the engagement members of the needle carrier has two sloped guiding surfaces.

13. The safety hypodermic syringe as in claim 1, wherein the positioning members of the plunger may engage with the positioning members of the needle carrier, and a user may rotate the plunger and the needle carrier in the opposite direction to disengage the engagement members of the needle carrier from the engagement members of the barrel.

14. The safety hypodermic syringe as in claim 1, wherein a break-apart member is provided in the proximal end of the fluid space and has an inner diameter slightly smaller than the outer diameter of the needle carrier so that a user may move the plunger to the right and to the left for several times to break a neck portion apart from the rest of the plunger and a circular bung may remain inside the barrel to seal off the barrel.

15. The safety hypodermic syringe as in claim 1, wherein the diameter of the needle cap is slightly larger than that of the hole so that a user may insert the needle cap in an inverted manner into the hole to block the hole.
